# DEMANDE DE BREVET EUROPEEN

(11) **EP 0 765 655 A1**
(43) Date de publication de la demande: **02.04.1997**
(21) Numéro de dépôt: 96401758.6
(22) Date de dépôt: 08.08.1996
(51) Int. Cl.: A61K 7/09

(54) **Nouveau procédé pour la déformation permanente des matières kératiniques**

(30) Priorité: 18.09.1995 FR 9510914
(71) Demandeur: L'OREAL, 75008 Paris (FR)
(72) Inventeur: Franbourg, Alain, 75010 Paris (FR); Lopez, Michel Angel, 93340 Le Raincy (FR); Leroy, Frédéric, 92210 Saint Cloud (FR); Malle, Gérard, 47-49, Avenue Clémenceau, 77100 Meaux (FR)
(74) Mandataire: Andral, Christophe André Louis

(57) **Abrégé**

L'invention concerne un procédé de traitement des matières kératiniques, en particulier des cheveux, en vue d'obtenir une déformation permanente de ces dernières, en particulier sous la forme de cheveux permanentés, ledit procédé étant caractérisé par le fait qu'il comprend les étapes suivantes : (i) on applique sur la matière kératinique à traiter une composition réductrice, les moyens (rouleaux) nécessaires à la mise sous tension mécanique de la matière kératinique étant mis en oeuvre avant, pendant ou après ladite application, (ii) puis on rince la matière kératinique ainsi traitée par une composition aqueuse comprenant au moins un polymère cationique, (iii) on applique sur la matière kératinique ainsi rincée une composition oxydante, la matière kératinique traitée étant séparée des moyens de mise sous tension utilisés à l'étape (i) avant ou après ladite application de la composition oxydante, (iv) et éventuellement on rince à nouveau la matière kératinique.

## Description

L'invention concerne un procédé de traitement des matières kératiniques, en particulier des cheveux, en vue d'obtenir une déformation permanente de ces dernières, en particulier sous la forme de cheveux permanentés.

On sait que la technique la plus usuelle pour obtenir une déformation permanente des cheveux, consiste, dans un premier temps, à réaliser l'ouverture des liaisons disulfures-S-S- de la kératine (cystine) à l'aide d'une composition contenant un agent réducteur (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique, l'acide thioglycolique ainsi que ses esters, notamment le monothioglycolate de glycérol.

A cet égard, et bien que possédant une odeur désagréable, l'acide thioglycolique est particulièrement efficace, et constitue ainsi le composé de référence en permanente pour réduire les liaisons disulfures de la kératine.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème de la technique des permanentes connues à ce jour est que leur application sur les cheveux induit à la longue une altération de la qualité des cheveux. Les causes essentiellles de cette altération de la qualité des cheveux sont une diminution de leur propriétés cosmétiques, telles que leur brillance, et une dégradation de leur propriétés mécaniques, plus particulièrement une dégradation de leur résistance mécanique due à un gonflement des fibres kératiniques lors du rinçage entre l'étape de réduction et l'étape d'oxydation qui peut également se traduire par une augmentation de leur porosité.

Pour résoudre ce problème d'altération de la qualité des cheveux, il a été proposé d'associer des polymères cationiques soit aux agents réducteurs, soit aux agents oxydants.

Mais, ces solutions se révèlent insatisfaisantes dans la mesure où elles ne résolvent pas le problème de la diminution des propriétés mécaniques des cheveux.

Il a été observé que lors de la phase de rinçage à l'eau des cheveux entre les deux étapes principales du procédé de permanente, il se produit une très forte augmentation du diamètre des fibres capillaires qui entraîne par la suite une dégradation mécanique irréversible de ces fibres.

Pour résoudre ce problème de gonflement des fibres, il a été proposé de rincer les fibres réduites par une solution saline concentrée ou par une solution présentant un pH acide. Cependant, les améliorations observées concernent principalement les propriétés cosmétiques des cheveux, les propriétés mécaniques ne sont que peu ou pas préservées.

La présente invention a notamment pour but de résoudre les problèmes ci-dessus.

Plus précisément, elle a pour but de proposer un nouveau procédé de traitement convenant à la déformation permanente des matières kératiniques et qui permet de limiter, voire d'empêcher, la dégradation des propriétés mécaniques des matières kératiniques due notamment au gonflement de ces dernières après un traitement de permanente.

Elle a également pour but de proposer un procédé tel que ci-dessus qui permette d'améliorer les propriétés cosmétiques, telles que la brillance, la douceur et la facilité de démêlage, des fibres kératiniques lorsque celles-ci subissent un traitement de déformation permanente.

Or, la demanderesse vient de découvrir que si l'on utilise des polymères cationiques dans une phase de rinçage intermédiaire, on obtient un excellent état de la fibre capillaire au terme du procédé de permanente. En particulier la conservation des propriétés mécaniques est remarquable et les variations dimensionnelles des fibres observées dans les traitements traditionnels sont fortement limitées, voire totalement supprimées.

Ainsi, la présente invention a pour objet un procédé de traitement des matières kératiniques, en particulier des cheveux, en vue d'obtenir une déformation permanente de ces dernières, en particulier sous la forme de cheveux permanentés, ledit procédé étant caractérisé par le fait qu'il comprend les étapes suivantes : (i) on applique sur la matière kératinique à traiter une composition réductrice, les moyens (rouleaux) nécessaires à la mise sous tension mécanique de la matière kératinique étant mis en oeuvre avant, pendant ou après ladite application, (ii) puis on rince la matière kératinique ainsi traitée par une composition aqueuse comprenant au moins un polymère cationique, (iii) on applique sur la matière kératinique ainsi rincée une composition oxydante, la matière kératinique traitée étant séparée des moyens de mise sous tension utilisés à l'étape (i) avant ou après ladite application de la composition oxydante, (iv) et éventuellement on rince à nouveau la matière kératinique.

Le procédé selon l'invention convient particulièrement bien à l'obtention d'une chevelure permanentée.

Après l'étape (ii) et avant l'étape (iii) du procédé selon l'invention, il est possible d'ajouter une étape de rinçage généralement à l'eau, bien que celle-ci ne s'avère pas nécessaire.

L'efficacité du procédé selon l'invention présente notamment l'avantage d'être indépendant du pH de la solution comprenant ledit polymère cationique.

D'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

La concentration en polymères cationiques tels que définis ci-dessus dans la composition aqueuse est avantageusement comprise entre 0,1 et 5% en poids par rapport au poids de ladite composition.

Dans la présente demande, l'expression "polymère cationique" désigne un polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne polymère, soit être portés par un substituant latéral.

Les polymères cationiques utilisés ont de préférence une masse moléculaire comprise entre 10³ et 3.10⁶ environ.

Les polymères cationiques utilisés sont en particulier ceux ayant un taux de quaternisation, exprimé en équivalents cationiques par gramme de polymère, d'au moins 0,05 méq cationique/g. On utilise notamment des polymères cationiques qui contiennent par exemple au moins 10 % en poids de motifs comportant des groupements amine ou ammonium quaternaire.

Lorsque le polymère cationique contient des groupements amine ou ammonium quaternaire portés par un substituant latéral, la chaîne polymère est par exemple une chaîne acrylique, vinylique, siliconée, fluorée ou sacharidique.

Parmi les polymères cationiques, on peut citer plus particulièrement les protéines (ou hydrolysats de protéines) quaternisées, les polysiloxanes quaternisés et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 000 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate";
- les hydrolysats de collagène portant des groupements chlorure de triméthylammonium et de triméthylstéarylammonium, vendus sous la dénomination de "Quat-Pro S" par la Société Maybrook et dénommnés dans le dictionnaire CTFA "Steartrimonium Hydrolyzed Collagen";
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule : 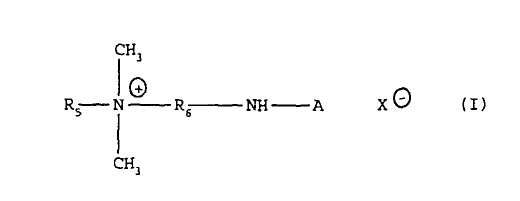 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominatioons "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Une autre famille de polymères cationiques est celle des polymères cationiques siliconés. Parmi ces polymères, ont peut citer :
(a) les polysiloxanes quaternisés dénommés dans le dictionnaire CTFA "amodiméthicone" et répondant à la formule : 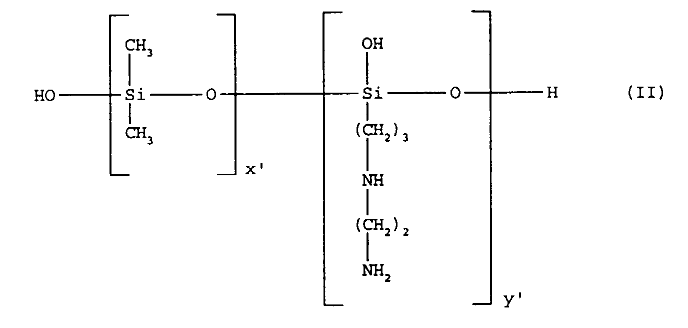 dans laquelle x' et y' sont des nombres entiers dépendant du poids moléculaire, généralement tels que ledit poids moléculaire est compris entre 5 000 et 10 000 environ ;
(b) les polymères cationiques siliconés répondant à la formule :

   R'ₐG₃₋ₐ-Si(OSiG₂)ₙ-(OSiG_{b}R'_{2-b})ₘ-O-SiG₃₋ₐ-R'ₐ (III)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10;
   R' est un radical monovalent de formule -C_{q}H_{2q}L dans laquelle q est un nombre de 2 à 8 et L est un groupement aminé éventuellement quaternisé choisi parmi les groupements :
      -NR"-CH₂-CH₂-N'(R")₂
      -N(R")₂
      -N^{⊕}(R")₃A⁻
      -N^{⊕}(R")₃ A⁻
      -N^{⊕}(R")₃A⁻
      -N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Un produit correspondant à cette définition est le polymère dénommé "triméthylsilylamodiméthicone", répondant à la formule : 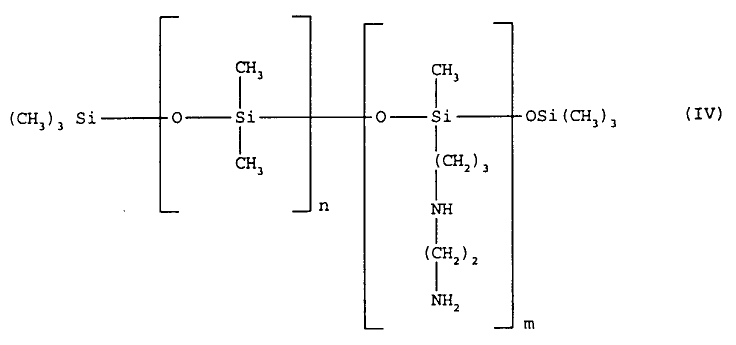 dans laquelle n et m ont les significations données ci-dessus (cf formule III).
   De tels polymères sont décrits par exemple dans la demande de brevet EP-A-95238.
(c) les polymères cationiques siliconés répondant à la formule : 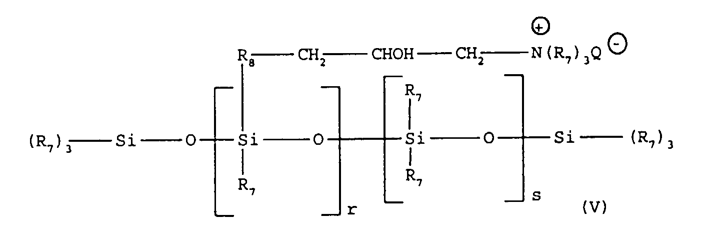 dans laquelle
   R₇ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₈ représente un radical hydrocarboné divalent, noatmment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels polymères sont décrits plus particulièrement dans le brevet US 4 185 087.

Un polymère entrant dans cette classe est le polymère vendu par la Société Union Carbide sous la dénomination "Ucar Silicone ALE 563.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "Emulsion Cationique DC 929" par la Société Dow Corning qui comprend, outre l'amodiméthicone, un agent de surface cationiques comprenant un mélange de produits répondant à la formule : 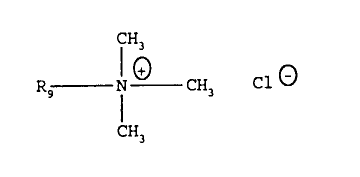 dans lequel R₉ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

Un autre produit commercial utilisable selon l'invention est le produit vendu sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40
dénommé encore octoxynol-40,
un autre agent de surface non ionique de formule : C₁₂H₂₅-(OCH₂-CH₂)ₙ-OH où n = 6 encore dénommé isolaureth-6,
et du glycol.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits vendus sous la dénomination "Gafquat" par la Société GAF Corporation, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternairees d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 et plus particulièrement le produit commercialisé sous la dénomination "Jaguar C.13 S" vend par la Société Meyhall.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclohomopolymères de méthyl diallyl amine ou de diméthyl diallyl ammonium tels que les homopolymères comportant comme constituant principal de la chaîne des motifs répondant aux formules (VI) ou (VI') : 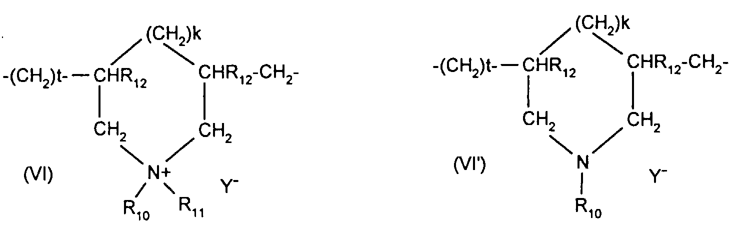 formules dans lesquelles k et t sont égaux à 0 ou 1, la somme k + t étant égale à 1 ; R12 désigne un atome d'hydrogène ou un radical méthyle ; R10 et R11, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R10 et R11 peuvent désigner conjointement avec l'atome d'azote auquel ils sont rattachés, des groupement hétérocycliques, tels que pipéridinyle ou morpholinyle ; γ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Par groupement amidoalkyle inférieur, on entend, au sens de la présente invention, un groupement amidoalkyle ayant de 1 à 6 atomes de carbone.
   Parmi les polymères définis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurrents répondant à la formule : 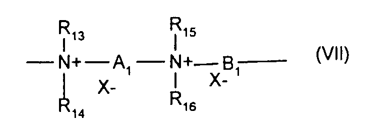 formule (VII) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2-;
   d) un groupement uréylène de formule : -NH-CO-NH- ;
   De préférence, X⁻ est un anion tel que le chlorure ou le bromure.
   Ces polymères ont une masse moléculaire généralement comprise entre 1000 et 100000.
   Par radicaux hydroxyalkylaliphatiques inférieurs, on entend, au sens de la présente invention, des radicaux hydroxyalkylaliphatiques ayant de 1 à 6 atomes de carbone.
   Des polymères de ce type sont notamment décrits dans les brevets français 2.320.330, 2.270.846, 2.316.271, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 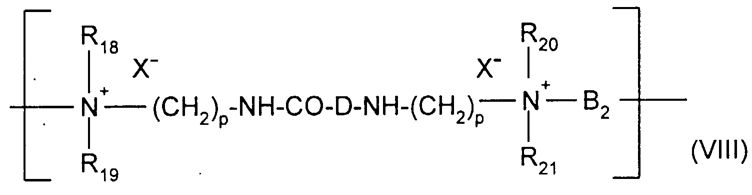 dans laquelle :
   - R18, R19, R20 et R21, identiques ou différents, représentent un atome d'hydrogène, le radical méthyle, éthyle, propyle, β-hydroxyéthyle, β-hydroxypropyle ou le radical -CH2CH2(OCH2CH2)qOH, q étant un nombre entier de 0 à 6, R1 8, R19, R20 et R21 n'étant pas simultanément l'atome d'hydrogène,
   - B2 désigne un groupement polyméthyléniques contenant de 2 à 20 atomes de carbone pouvant être linéaire ou ramifié, saturé ou insaturé, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, de préférence B2 désigne un radical de formule -CH2CH2OCH2CH2-,
   - p désigne un nombre entier variant de 1 à 6 environ,
   - D peut être nul ou peut représenter un groupement -(CH₂)ᵣ-CO- dans lequel r désigne un nombre égal à 4 ou à 7,
   - X⁻ est un anion dérivé d'un acide minéral ou organique.
   La masse moléculaire de ces polymères est généralement inférieure à 100 000, de préférence inférieure ou égale à 50 000,
   De tels polymères peuvent être préparés selon les procédés décrits dans les brevets US 4 157 388, 4 390 689, 4 702 906 et 4 719 282.
   On peut par exemple citer parmi ceux-ci, les produits "MIRAPOL AD1", "MIRAPOL AZ1" et "MIRAPOL A-15", "MIRAPOL-9", "MIRAPOL-175" et "MIRAPOL-95" vendus par la société Miranol.
   Ces polymères ci-dessus correspondent aux polymères de formule (VIII) dans laquelle :
   i) R18, R19, R20, R21 représentent le radical méthyle, B2 le radical de formule CH2CH2OCH2CH2-, D représente un groupement -(CH₂)₄-CO- et X désigne un atome de chlore ; un polymère de ce type est vendu par la société MIRANOL sous le nom de MIRAPOL-AD1,
   ii) R18, R19, R20, R21 représentent le radical méthyle, B2 le radical de formule CH2CH2OCH2CH2-, D représente un groupement -(CH₂)₇-CO- et X désigne un atome de chlore ; un polymère de ce type est vendu par la société MIRANOL sous le nom de MIRAPOL-AZ1,
   iii) R18, R19, R20, R21 représentent le radical méthyle, B2 le radical de formule CH2CH2OCH2CH2-, D désigne la valeur zéro et X désigne un atome de chlore ; un polymère de ce type est vendu par la société MIRANOL sous le nom de MIRAPOL-A15,
   iiii) R18, R19, R20, R21 représentent le radical méthyle, B2 le radical de formule CH2CH2OCH2CH2-, les copolymères blocs formés de motifs correspondant aux polymères décrits aux alinéas i) et iii) ci-dessus, et vendus par la société MIRANOL sous les noms de MIRAPOL-9, MIRAPOL-175 et MIRAPOL-95.
(12) les homopolymères ou copolymères dérivés des acides acrylique ou méthacrylique et comportant des motifs : 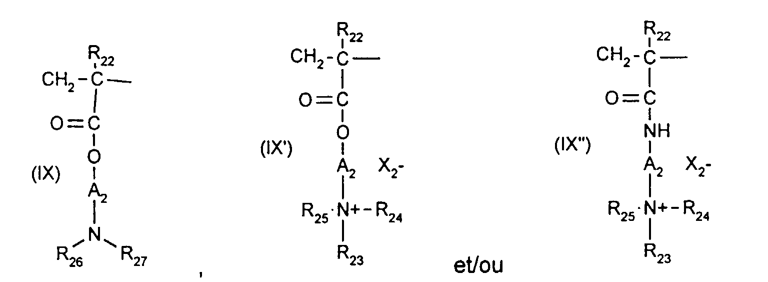 dans lesquels les groupements R22 désignent indépendamment H ou CH3, les groupements A₂ désignent indépendamment un groupe alcoyle linéaire ou ramifié de 1 à 6 atomes de carbone ou un groupe hydroxyalcoyle de 1 à 4 atomes de carbone,
   les groupements R23, R24, R25, identiques ou différents, désignant indépendamment un groupe alcoyle de 1 à 18 atomes de carbone ouun radical benzyle,
   les groupements R26 et R27 représentent un atome d'hydrogène ou un groupement alcoyle de 1 à 6 atomes de carbone,
   X₂⁻ désigne un anion, par exemple méthosulfate ou halogénure, tel que chlorure ou bromure.
   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyles inférieurs, des esters d'alcoyles, des acides acrylique ou méthacrylique, la vinylpyrrolidone ou des esters vinyliques.
   Par alcoyles inférieurs, on entend, au sens de la présente invention, des alcoyles ayant de 1 à 6 atomes de carbone.
   Parmi ces composés, on peut citer en particulier le composé de formule (IX") dans laquelle R22, R24 et R25 représentent le radical méthyle, R23 le radical éthyle, A2 le radical de formule -(CH2)3- et X₂⁻ l'anion bromure.
(13) Les polymères quaternaires de vinylpyrrolidone et de vinylimidazole tels que par exemple les produits commercialisés sous les dénominations Luviquat FC 905, FC 550 et FC 370 par la société B.A.S.F..

Parmi ces polymères cationiques, on utilise de préférence les polymères de formules (VI), (VI'), (VII) et (VIII), et plus particulièrement les polymères choisis parmi le composé de formule (VII) dans laquelle R13, R14, R15 et R16 représentent le radical méthyle, A1 représente le radical de formule -(CH2)3-, B1 représente le radical de formule -(CH2)6-et X⁻ représente l'anion chlorure (nommé ultérieurement Mexomère PO), le composé de formule (VII) dans laquelle R13 et R14 représentent le radical éthyle, R15 et R16 représentent le radical méthyle, A1 et B1 représentent le radical de formule -(CH2)3- et X⁻ représente l'anion bromure (nommé ultérieurement Mexomère PAK), le composé de formule (VII) dans laquelle R13, R14, R15 et R16 représentent le radical méthyle, A1 et B1 représentent le radical de formule -(CH2)3- et X⁻ représente l'anion chlorure, le composé de formule (VII) dans laquelle R13, R14, R15 et R16 représentent le radical méthyle, A1 et B1 représentent le radical de formule -(CH2)6- et X⁻ représente l'anion chlorure, le composé de formule (VII) dans laquelle R13, R14, R15 et R16 représentent le radical méthyle, A1 représente le radical de formule -(CH2)3-, B1 représente le radical de formule -(CH2)9- et X⁻ représente l'anion chlorure, le composé de formule (VII) dans laquelle R13, R14, R15 et R16 représentent le radical méthyle, A1 et B1 représentent le radical de formule -(CH2)6- et X⁻ représente l'anion bromure, le composé de formule (VII) dans laquelle R13, R14, R15 et R16 représentent le radical méthyle, A1 représente le radical de formule -(CH2)3-, B1 représente le radical de formule -(CH2)2-O-(CH2)2- et X⁻ représente l'anion chlorure, le composé de formule (VII) dans laquelle R13, R14, R15 et R16 représentent le radical méthyle, A1 représente le radical de formule -(CH2)3-, B1 représente le radical de formule -(CH2)2-O-(CH2)2-O-(CH2)2- et X⁻ représente l'anion chlorure, le composé de formule (VII) dans laquelle R13, R14, R15 et R16 représentent le radical méthyle, A1 représente le radical de formule -(CH2)2-O-(CH2)2-, B1 représente le radical de formule -(CH2)2-O-(CH2)2-O-(CH2)2- et X⁻ représente l'anion chlorure, le MIRAPOL A-15 (dénommé polyquaternium-2 dans le dictionnaire CTFA), le MIRAPOL AD-1 (dénommé polyquaternium-17 dans le dictionnaire CTFA), le MIRAPOL AZ-1 (dénommé polyquaternium-18 dans le dictionnaire CTFA), le MIRAPOL 175 (dénommé polyquaternium-27 dans le dictionnaire CTFA) et le MERQUAT 100.

La composition aqueuse mise en oeuvre à l'étape (ii) du procédé selon l'invention peut se présenter sous la forme d'une lotion, épaissie ou de préférence non épaissie, d'une crème, d'un gel ou de toute autre forme appropriée.

Ainsi, le véhicule de cette composition est de préférence de l'eau, mais il peut être également une solution hydroalcoolique d'un alcool inférieur tel que l'éthanol, l'isopropanol ou le butanol.

La composition aqueuse mise en oeuvre à l'étape (ii) du procédé selon l'invention peut bien entendu contenir d'autres composés, tels que des protéines ou des acides aminés.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Dans la première étape indispensable du procédé selon l'invention, on applique sur les cheveux une composition réductrice, ladite composition réductrice contenant au moins un agent actif approprié pour la réduction des liaisons disulfures de la kératine. Cette application peut être réalisée avant, pendant ou après l'habituelle étape de mise sous tension des cheveux.

Parmi les agents actifs appropriés pour la réduction des liaisons disulfures de la kératine, on peut citer des sulfites, des bisulfites, des alkyl-phosphines ou, de préférence, des thiols. Parmi ces derniers, ceux préférentiellement utilisés sont choisis parmi l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les esters de cystéine, tels que le cystéïnate de glycérol, les N-mercaptoalkylamides de sucres tels que le N-(mercapto-2-éthyl)gluconamide, l'acide thiolactique et ses esters tels que le monothiolactate de glycérol, l'acide 3-mercaptopropionique et ses esters, tels que le 3-mercaptopropionate de glycérol, l'acide thiomalique, l'acide 2-hydroxy 3-mercaptopropionique et ses esters, tels que le 2-hydroxy 3-mercaptopropionate de glycérol, la panthétéine, le thioglycérol, les sulfites ou les bisulfites d'un métal alcalin ou alcalino-terreux, les N-(mercaptoalkyl)ω-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448, les N-mercaptoalkyl alcanediamides décrits dans la demande de brevet EP-A-653.202..

On utilise de préférence l'acide thioglycolique, l'acide thiolactique, la cystéïne et ses divers dérivés, la cystéamine et ses dérivés, l'acide 3-mercaptopropionique, ainsi que leurs esters ou leurs sels, notamment le monothioglycolate de glycérol.

Ces agents actifs peuvent être utilisés seuls ou en mélange.

Lorsque l'on utilise l'acide thioglycolique, l'acide thiolactique, l'acide 3-mercaptopropionique, l'acide 2-hydroxy 3-mercaptopropionique, la cystéine ou la cystéamine, ou l'un de leurs sels ou de leurs dérivés, à titre d'agent réducteur, le pH de l'ensemble de la composition selon l'invention est de préférence compris entre 6 et 11,5 et encore plus préférentiellement entre 7 et 10.

Lorsque l'on utilise les esters de l'acide thioglycolique ou de l'acide thiolactique ou de l'acide 3-mercaptopropionique, de la cystéïne ou de l'acide 2-hydroxy 3-mercaptopropionique, à titre d'agent réducteur, le pH de l'ensemble de la composition selon l'invention est de préférence compris entre 5 et 10 et encore plus préférentiellement entre 6 et 9.

Les agents réducteurs mentionnés ci-avant sont généralement présents à une concentration qui peut être comprise entre 1 et 20 % en poids par rapport au poids total de la compositon réductrice.

Les pH des compositions réductrices peuvent être ajustés classiquement par ajout d'agents basifiants, tels que par exemple la soude, l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires, ou un carbonate organique tel que le carbonate de guanidine, tous ces composés pouvant bien entendu être pris seuls ou en mélange. Comme indiqué précédemment, l'un des grands avantages du procédé selon l'invention est qu'il convient parfaitement dans le cas des compositions réductrices carbonatées. La concentration en ion carbonate CO₃ ²⁻ dans la composition réductrice est alors généralement comprise entre 0,1 et 1,6 M.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl) ω-hydroxyalkyl-amides décrits dans la demande de brevet EP-A-354 835, les disulfures des N-mono ou N,N-dialkylmercapto-4 butyramides décrits dans la demande de brevet EP-A-368 763, les disulfures des aminomercaptoalkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A465 342, les disulfures des alkylaminomercaptoalkylamides décrits dans la demande de brevet EP-A-514 282 et les disulfures des N-mercaptoalkyl alcane diamides décrits dans la demande de brevet EP-A-653.202. Ces disulfures sont généralement présents dans un rapport molaire de 0,5 à 2,5, et de préférence de 1 à 2, par rapport à l'agent réducteur (voir brevet US 3 768 490).

Avant de procéder à l'étape suivante de rinçage, il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur les cheveux. Cette phase d'attente est effectuée généralement en laissant reposer à l'air libre (température ambiante) la chevelure traitée, mais elle peut être également effectuée à une température plus élevée. Pendant cette phase d'attente, on prend soin que les cheveux ne sèchent pas complètement et restent humides jusqu'au moment de la mise en oeuvre de l'étape suivante (à cet effet, utilisation possible de bonnets, de gels de protection par exemple).

Dans la deuxième étape du procédé (étape (ii)), les cheveux imprégnés de la composition réductrice sont donc ensuite rincés soigneusement par la composition aqueuse décrite ci-dessus.

Puis, dans une troisième étape (étape (iii)), on applique sur les cheveux ainsi rincés une composition oxydante dans le but de fixer la nouvelle forme imposée aux cheveux.

La composition oxydante contient un agent oxydant qui peut être choisi parmi l'eau oxygénée, un bromate alcalin, un persel ou un polythionate ou leur mélange, tel qu'un mélange de bromate alcalin et d'un persel.

La concentration en eau oxygénée peut varier de 1 à 10 volumes, mais est de préférence de 8 volumes, la concentration en bromate alcalin est généralement de 1 à 12 % et celle en persel de 0,1 et 15 % en poids par rapport au poids total de la composition oxydante.

Le pH de la composition oxydante est généralement compris entre 2 et 10.

La composition oxydante peut contenir des additifs cosmétiques bien connus pour ce type de composition.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine.

La composition oxydante peut également contenir des agents alcalinisants ou acidifiants, des agents conservateurs, des agents séquestrants, des opacifiants et éventuellement un polymère cationique tel que défini ci-dessus pour la composition aqueuse.

On peut retirer de la chevelure les moyens mécaniques (rouleaux, bigoudis et analogues) qui maintenaient sous tension et dans la forme désirée les cheveux tout au long du traitement avant ou après l'étape de fixation.

Enfin, dans l'éventuelle étape du procédé selon l'invention (étape (iv)), les cheveux imprégnés de la composition oxydante sont rincés soigneusement, généralement à l'eau.

On obtient finalement une chevelure présentant de bonnes propriétés cosmétiques : les cheveux sont plus brillants, plus doux et plus faciles à démêler et de bonnes propriétés mécaniques .

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec le dos ou les dents d'un peigne ou à la main. Pendant le temps de pose de 5 à 60 minutes, en particulier de 5 à 30 minutes, on procède alors à un ou plusieurs nouveaux lissages puis on rince soigneusement au moyen de la composition aqueuse décrite précédemment et on applique une composition oxydante (ou fixatrice) telle que définie ci-dessus, que l'on laisse agir pendant environ 2 à 10 minutes puis on rince abondamment les cheveux.

Des exemples concrets illustrant l'invention vont maintenant être donnés.

### Exemple 1

### Etude comparative des propriétés mécaniques dynamiques des cheveux au cours d'un procédé de permanente

Les cheveux sont réduits à l'aide d'une composition réductrice contenant de l'acide thioglycolique à 7% en poids et ajustée à pH 8 par de l'ammoniaque.

Après avoir laissé agir la composition pendant environ 15 min., on rince avec l'une des compositions aqueuses de formulations suivantes
Formulation A (comparatif) :
   eau osmosée
Formulation B (comparatif) :
   tampon citrate pH 4,5
Formulation C (comparatif) :
   solution d'acide chlorhydrique (10⁻³M) de pH 3,0
Formulation D (comparatif) :
   solution d'acide chlorhydrique de pH 4,5
Formulation E (comparatif) :
   solution de chlorure de sodium à 8% en poids de pH spontané
Formulation F (comparatif) :
   solution de chlorure d'ammonium de pH 8
Formulation G (selon l'invention) :
   solution aqueuse de Mexomère PO à 1% en poids et de pH sontané
Formulation H (selon l'invention) :
   solution aqueuse de Mexomère PO à 1% en poids de pH 8
Formulation I (selon l'invention) :
   solution aqueuse de Mirapol 175 à 1% en poids de pH spontané (pH 7)

Puis, on applique la composition oxydante contenant 6% en poids d'eau oxygénée présentant un pH de 3 ajusté par de l'acide chlorhydrique.

On laisse agir la composition oxydante pendant environ 10 minutes, puis on rince abondamment les cheveux à l'eau.

Après séchage, on évalue l'augmentation en pourcentage du module d'élasticité des cheveux à la fin du traitement par rapport à la fin du rinçage intermédiaire.

Le mode opératoire est le suivant :

Des cheveux de longueur de 10 mm sont montés entre les mors d'un appareil viscoélasticimètre de marque METRAVIB® permettant de mesurer le module d'élasticité (N/m) de ces cheveux, ces mors étant placés dans une chambre pouvant être remplie d'un liquide. Les cheveux sont ensuite soumis à une tension statique de 2% et à une déformation dynamique d'amplitude maximale de 0,2% à 15,6 Hz.

On applique à ces cheveux le traitement indiqué ci-dessus (réduction, rinçage intermédiaire et oxydation), le rinçage intermédiaire étant réalisé avec les différentes formulations A à I.

Grâce à l'appareillage qui possède un capteur de force, on évalue l'augmentation en pourcentage du module de raideur des cheveux à la fin du traitement par rapport à la fin du rinçage intermédiaire.

Les résultats sont rassemblés dans le tableau 1 suivant.

**Tableau 1**

| Composition de rinçage | augmentation du module de raideur (%) |
|---|---|
| Formulation A | 36,0 |
| Formulation B | 38,2 |
| Formulation C | 13,6 |
| Formulation D | 43,1 |
| Formulation E | 69,8 |
| Formulation F | 37,8 |
| Formulation G | 113,3 |
| Formulation H | 126,3 |
| Formulation I | 93,5 |

II a déjà été décrit qu'après le rinçage intermédiaire on observe une chute du module d'élasticité des cheveux préalablement réduits (M.L. Garcia, E.M. Nadgorny, L.J. Wolfram, J. Soc. Cosmet. Chem., 41 (2), 149, (1990)). On sait aussi que ce module remonte lors de l'étape d'oxydation (de fixation), mais qu'il ne retrouve jamais après cette étape d'oxydation sa valeur initiale.

Ainsi, selon le tableau 1, on s'aperçoit clairement que l'augmentation du module de raideur des cheveux traités est la plus élevée lorsque le rinçage intermédiaire est réalisé avec des compositions selon l'invention.

### Exemple 2

On prépare une composition réductrice de déformation permanente des cheveux en procédant au mélange des composés suivants :

| | |
|---|---|
| Acide thioglycolique | 9g |
| Acide thiolactique | 2g |
| Carbonate d'ammonium | 6g |
| Ammoniaque qsp | pH8,4 |
| Eau déminéralisée qsp | 100g |

Cette composition est appliquée sur des cheveux mouillés préalablement enroulés sur des rouleaux de mise en plis.

Après avoir laissé agir la composition pendant environ 15 mminutes, on rince soigneusement la chevelure avec une solution aqueuse à 1% en poids de poly[dichlorure de (diméthyliminio)1,3-propanediyl(diméthyliminio)-1,6hexanediyle] dénommé Mexomère PO, à son pH spontané. Puis on applique la composition oxydante suivante :

| | |
|---|---|
| Eau oxygénée à 200 volumes | 4,8g |
| Sulfate d'hydroxy-8-quinoléïne | 0,01g |
| Phénacétine | 0,05g |
| Acide citrique qsp | pH3 |
| Eau déminéralisée qsp | 100g |

On laisse agir la composition oxydante 10 minutes puis on enlève les rouleaux et rince abondamment la chevelure à l'eau.

Après séchage sous casque, les cheveux présentent de belles boucles très régulières et nerveuses qui se démêlent particulièrement bien à sec.

## Revendications

1. Procédé de traitement des matières kératiniques, en particulier des cheveux, en vue d'obtenir une déformation permanente de ces dernières, en particulier sous la forme de cheveux permanentés, ledit procédé étant caractérisé par le fait qu'il comprend les étapes suivantes : (i) on applique sur la matière kératinique à traiter une composition réductrice, les moyens (rouleaux) nécessaires à la mise sous tension mécanique de la matière kératinique étant mis en oeuvre avant, pendant ou après ladite application, (ii) puis on rince la matière kératinique ainsi traitée par une composition aqueuse comprenant au moins un polymère cationique, (iii) on applique sur la matière kératinique ainsi rincée une composition oxydante, la matière kératinique traitée étant séparée des moyens de mise sous tension utilisés à l'étape (i) avant ou après ladite application de la composition oxydante, (iv) et éventuellement on rince à nouveau la matière kératinique.

2. Procédé selon la revendication précédente, caractérisé en ce que la concentration en polymères cationiques dans la composition aqueuse de l'étape (ii) est comprise entre 0,1 et 5% en poids par rapport au poids de ladite composition.

3. Procédé selon l'une des revendications précédentes, caractérisé en ce que le polymère cationique est choisi parmi ceux qui contiennent des motifs comportant des groupements amine primaires, secondaires, tertiaires et/ou quaternaires pouvant soit faire partie de la chaîne polymère, soit être portés par un substituant latéral.

4. Procédé selon l'une quelconque des revendications précédentes, caractérisé en ce que le polymère cationique est choisi parmi ceux ayant un taux de quaternisation, exprimé en équivalents cationiques par gramme de polymère, d'au moins 0,05 méq cationique/g.

5. Procédé selon l'une quelconque des revendications précédentes, caractérisé en ce que le polymère cationique est choisi parmi les protéines (ou hydrolysats de protéines) quaternisées, les polysiloxanes quaternisés et les polymères du type polyamine, polyaminoamide et polyammonium quaternaire.

6. Procédé selon la revendication précédente, caractérisé en ce que le polymère cationique est choisi parmi les polymères du type polyamine, polyaminoamide et polyammonium quaternaire.

7. Procédé selon la revendication 6, caractérisé en ce que les polymères du type polyamine, polyaminoamide et polyammonium quaternaire sont choisis parmi les cyclohomopolymères de méthyl diallyl amine ou de diméthyl diallyl ammonium, tels que les homopolymères comportant comme constituant principal de la chaîne des motifs répondant aux formules (VI) ou (VI') : 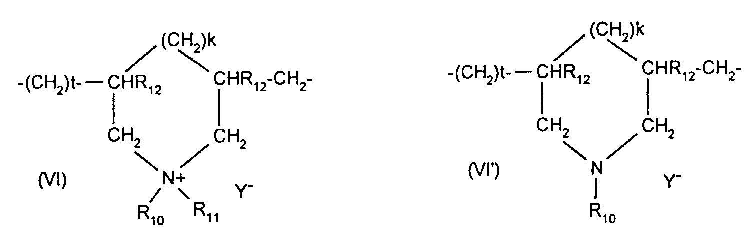 formules dans lesquelles k et t sont égaux à 0 ou 1, la somme k + t étant égale à 1 ; R12 désigne un atome d'hydrogène ou un radical méthyle ; R10 et R11, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R10 et R11 peuvent désigner conjointement avec l'atome d'azote auquel ils sont rattachés, des groupement hétérocycliques, tels que pipéridinyle ou morpholinyle ; Y⁻ est un anion.

8. Procédé selon la revendication 6, caractérisé en ce que les polymères du type polyamine, polyaminoamide et polyammonium quaternaire sont choisis parmi les polymère de diammonium quaternaire contenant des motifs récurrents répondant à la formule : 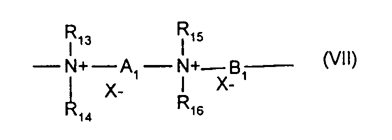 formule (VII) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique, de préférence, X⁻ est un anion tel que le chlorure ou le bromure;
A1, R13 et R15 peuvent former avec les deux atomes d'azote auxquels ils sont rattachés un cycle pipérazinique ; en outre si A1 désigne un radical alkylène ou hydroxyalkyléne linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2-;
d) un groupement uréylène de formule : -NH-CO-NH- ;

9. Procédé selon la revendication 6, caractérisé en ce que les polymères du type polyamine, polyaminoamide et polyammonium quaternaire sont choisis parmi les polymères de polyammonium quaternaires constitués de motifs de formule (VIII): 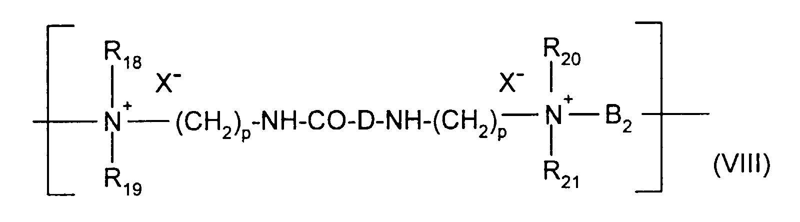 dans laquelle :
- R18, R19, R20 et R21, identiques ou différents, représentent un atome d'hydrogène, le radical méthyle, éthyle, propyle, β-hydroxyéthyle, β-hydroxypropyle ou le radical -CH2CH2(OCH2CH2)qOH, q étant un nombre entier de 0 à 6, R18, R19, R20 et R21 n'étant pas simultanément l'atome d'hydrogène,
- B2 désigne un groupement polyméthyléniques contenant de 2 à 20 atomes de carbone pouvant être linéaire ou ramifié, saturé ou insaturé, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, de préférence B2 désigne un radical de formule -CH2CH2OCH2CH2-,
- p désigne un nombre entier variant de 1 à 6 environ,
- D peut être nul ou peut représenter un groupement -(CH₂)ᵣ-CO-, dans lequel r désigne un nombre égal à 4 ou à 7,
- X- est un anion dérivé d'un acide minéral ou organique.

10. Procédé selon l'une des revendications 6 à 10, caractérisé en ce que les polymères du type polyamine, polyaminoamide et polyammonium quaternaire sont choisis parmi le composé de formule (VII) dans laquelle R13, R14, R15 et R16 représentent le radical méthyle, A1 représente le radical de formule -(CH2)3-, B1 représente le radical de formule -(CH2)6- et X⁻ représente l'anion chlorure, le composé de formule (VII) dans laquelle R13 et R14 représentent le radical éthyle, R15 et R16 représentent le radical méthyle, A1 et B1 représentent le radical de formule -(CH2)3- et X⁻ représente l'anion bromure, le composé de formule (VII) dans laquelle R13, R14, R15 et R16 représentent le radical méthyle, A1 et B1 représentent le radical de formule -(CH2)3- et X⁻ représente l'anion chlorure, le composé de formule (VII) dans laquelle R13, R14, R15 et R16 représentent le radical méthyle, A1 et B1 représentent le radical de formule -(CH2)6- et X⁻ représente l'anion chlorure, le composé de formule (VII) dans laquelle R13, R14, R15 et R16 représentent le radical méthyle, A1 représente le radical de formule -(CH2)3-, B1 représente le radical de formule -(CH2)9- et X⁻ représente l'anion chlorure, le composé de formule (VII) dans laquelle R13, R14, R15 et R16 représentent le radical méthyle, A1 et B1 représentent le radical de formule -(CH2)6- et X⁻ représente l'anion bromure, le composé de formule (VII) dans laquelle R13, R14, R15 et R16 représentent le radical méthyle, A1 représente le radical de formule -(CH2)3-, B1 représente le radical de formule -(CH2)2-O-(CH2)2- et X⁻ représente l'anion chlorure, le composé de formule (VII) dans laquelle R13, R14, R15 et R16 représentent le radical méthyle, A1 représente le radical de formule -(CH2)3-, B1 représente le radical de formule -(CH2)2-O-(CH2)2-O-(CH2)2- et X⁻ représente l'anion chlorure, le composé de formule (VII) dans laquelle R13, R14, R15 et R16 représentent le radical méthyle, A1 représente le radical de formule -(CH2)2-O-(CH2)2-, B1 représente le radical de formule -(CH2)2-O-(CH2)2-O-(CH2)2- et X⁻ représente l'anion chlorure, le MIRAPOL A-15®, le MIRAPOL AD-1®, le MIRAPOL AZ-1®, le MIRAPOL 175® et le MERQUAT 100®.

11. Procédé selon l'une quelconque des revendications précédentes, caractérisé en ce que la composition réductrice de l'étape (i) contient au moins un agent actif approprié pour la réduction des liaisons disulfures de la matière kératinique choisi parmi l'acide thioglycolique, l'acide thiolactique, la cystéïne et ses dérivés, la cystéamine et ses dérivés, l'acide 3-mercaptopropionique, l'acide 2-hydroxy 3-mercaptopropionique ainsi que leurs esters ou leurs sels.

12. Procédé selon l'une quelconque des revendications précédentes, caractérisé en ce que la composition réductrice de l'étape (i) contient au moins un agent actif approprié pour la réduction des liaisons disulfures de la matière kératinique associé à au moins un disulfure.

13. Procédé selon l'une quelconque des revendications précédentes, caractérisé en ce que la composition oxydante de l'étape (iii) contient un agent oxydant choisi parmi l'eau oxygénée, un bromate alcalin, un persel ou un polythionate ou leur mélange.
